# FASCICULE DE BREVET EUROPEEN

(11) **EP 2 635 669 B1**
(45) Date de publication et mention de la délivrance du brevet: **17.06.2015**
(21) Numéro de dépôt: 10795419.0
(22) Date de dépôt: 04.11.2010
(51) Int. Cl.: C12M 1/12, B01L 3/00

(54) **DISPOSITIF ET PROCEDE POUR ISOLER ET CULTIVER DES CELLULES VIVANTES SUR FILTRE OU EXTRAIRE LEUR MATERIEL GENETIQUE**
VORRICHTUNG UND VERFAHREN ZUR ISOLIERUNG UND KULTIVIERUNG LEBENDER ZELLEN AUF EINEM FILTER ODER ZUR EXTRAKTION DES GENETISCHEN MATERIALS DIESER ZELLEN
DEVICE AND METHOD FOR ISOLATING AND CULTIVATING LIVING CELLS ON A FILTER OR EXTRACTING THE GENETIC MATERIAL THEREOF FROM SAME

(43) Date de publication de la demande: 11.09.2013
(73) Titulaire: Screencell, 75013 Paris (FR)
(72) Inventeur: CAYRE, Yvon, F-75013 Paris (FR); BENALI-FURET, Naoual Linda, 95200 Sarcelles (FR); AUCANT, Cécile, 95200 Sarcelles (FR); WECHSEL, Janine, 95200 Sarcelles (FR)
(74) Mandataire: Maupilier, Didier
(86) Numéro de dépôt international: PCT/FR2010/052380
(87) Numéro de publication internationale: WO 2011/055091

(56) Documents cités:
- WO-A2-2009/106760
- FR-A1- 2 926 091
- FR-A5- 2 127 835
- US-A- 5 976 824
- US-A1- 2005 139 547
- US-A1- 2007 105 156

## Description

La présente invention concerne un dispositif et un procédé pour isoler et/ou cultiver des cellules vivantes ou fixées sur un filtre afin de procéder à toutes analyses cellulaires (cytologie, immunocytochimie, essais FISH, etc ...) ou extraire du matériel génétique éventuellement amplifié de cellules vivantes ou fixées isolées sur un filtre. Elle s'applique, en particulier, à isoler et/ou cultiver des cellules particulières présentes dans un liquide, notamment le sang, ou à extraire le matériel génétique de ces cellules particulières.

Certaines cellules particulières du sang, par exemple les cellules tumorales ou les trophoblastes, sont en très faible proportion et doivent être décomptées préalablement à des analyses cytologiques.

Il est connu d'appliquer un tampon de fixation à base de formaldéhyde à un échantillon sanguin pour fixer les cellules recherchées, puis de faire passer le liquide résultant dans un filtre poreux. Ce filtre est ensuite utilisé pour y examiner les cellules recherchées sous microscope, en laboratoire. Cependant, cette procédure ne permet pas l'obtention de cellules vivantes.

Or, l'inventeur a déterminé que l'obtention de cellules vivantes permettrait d'envisager l'identification de marqueurs spécifiques et d'appliquer dans de bonnes conditions des techniques de biologie moléculaire, de cytogénétique et de " FISH" (acronyme de " Fluorescence In Situ Hybridization ", pour hybridation fluorescente in situ) pour le diagnostic d'anomalies génétiques dans des cellules tumorales ou trophoblastiques.

On connaît, notamment des documents US 5 976 824, FR 2 127 835 et US 2007/105156, des dispositifs et méthodes pour recueillir un échantillon de cellules à partir d'un échantillon liquide, en mettant en oeuvre une aiguille adaptée à perforer un bouchon de tube à vide. Cependant, ces dispositifs et méthodes présentent des risques d'une piqûre accidentelle avec l'aiguille. De plus, ils ne permettent ni une mise en place aisée du tube à vide, ni son maintien de manière rigide pour éviter son retrait ou son déplacement qui permettrait à l'air d'entrer dans le tube à vide en passant à côté de l'aiguille dans le bouchon du tube à vide.

Le document FR2926091 décrit un dispositif pour isoler des cellules fixées ou vivantes sur un filtre ou extraire le matériel génétique de cellule. Il comporte un compartiment présentant un volume intérieur pour recevoir un liquide comportant les cellules, une ouverture inférieure et une entrée d'air, un piston mobile dans le compartiment et un filtre solidaire de l'ouverture du compartiment pour retenir les cellules lors du passage du liquide à travers le filtre.

Ce dispositif ne présente ni aiguille ni moyen d'aspiration.

La présente invention vise à remédier à ces inconvénients et à répondre à ce besoin en permettant de recueillir, dans des conditions compatibles avec des examens de laboratoire de routine, des cellules vivantes pouvant être subséquemment cultivées dans des milieux adaptés en présence de facteurs de croissance adéquats.

La présente invention concerne aussi l'extraction du matériel génétique éventuellement amplifié de cellules isolées sur filtre et la détection de mutations et des niveaux de l'expression de gènes de sensibilité et de résistance aux thérapies ciblées ou d'anomalies génétiques.

Elle s'applique, en particulier à recueillir et éventuellement amplifier de façon uniforme l'ADN ou l'ARN de cellules particulières présentes dans un liquide, notamment le sang.

In est connu, par exemple du document PCT/FR 2006/000562, d'appliquer un tampon de fixation à base de formaldéhyde à un échantillon sanguin pour fixer les cellules recherchées, puis de faire passer le liquide résultant dans un filtre poreux. Ce filtre est ensuite analysé en laboratoire pour y rechercher les cellules sous microscope. On peut, par la suite, les prélever sur le filtre pour des analyses, par exemple par une analyse génétique.

Cependant, cette procédure ne peut faire l'objet d'une reproduction à grande échelle et à un coût raisonnable, du fait du temps, des matériels et de la précision de travail qu'elle implique. Or cette reproduction à grande échelle et moindre coût permettrait la conduite d'analyses de biologie moléculaire à la fois sur des cellules tumorales et sur des cellules trophoblastiques. De plus, la fixation des cellules par le formaldéhyde ne permet pas d'obtenir du matériel génétique de bonne qualité : l'ADN est en partie dégradé et forme des ponts avec des protéines ambiantes et l'extraction d'ARN est pratiquement exclue.

La présente invention vise aussi à remédier à ces inconvénients et à répondre à ce besoin en permettant de recueillir dans des conditions compatibles avec des examens de laboratoire de routine, une grande proportion du matériel cellulaire, en particulier, ARN et ADN, des cellules considérées, en bon état. Il faut aussi noter que la présente invention permet l'isolement de cellules fixées à l'aide d'un fixateur contenant ou non du formaldéhyde.

A cet effet, selon un premier aspect, la présente invention vise un dispositif pour isoler des cellules fixées ou vivantes sur un filtre, caractérisé en ce qu'il comporte :
- un compartiment présentant un volume intérieur pour recevoir un liquide comportant lesdites cellules, une ouverture inférieure et une entrée d'air,
- un moyen mobile par rapport au compartiment, ce moyen mobile comportant des bras,
- un filtre solidaire, au moins temporairement, de ladite ouverture du compartiment, adapté à retenir lesdites cellules lors du passage du liquide à travers le filtre,
- une aiguille au moins temporairement solidaire de ladite ouverture du compartiment, de manière étanche, le filtre se trouvant entre l'aiguille et le volume intérieur du compartiment, ladite aiguille étant adaptée à perforer un bouchon de tube à vide présentant une dépression par rapport à la pression ambiante pour aspirer le liquide à travers ledit filtre et
- un moyen de liaison entre le compartiment et un cylindre de protection entourant l'aiguille et destiné à entourer, au moins partiellement, le tube à vide pendant l'aspiration du liquide à travers le filtre,
dans lequel le moyen mobile et le compartiment sont configurés pour être mis en mouvement relatif pour appliquer une force sur le filtre et libérer le filtre après aspiration du liquide à travers le filtre, et dans lequel les pattes du moyen mobile sont configurés pour, lorsque le doigt d'un opérateur met en mouvement relatif le moyen mobile et le compartiment, exercer une pression sur un support du filtre et libérer le support de filtre de l'ouverture du compartiment.

Ce dispositif permet ainsi l'isolement et la récupération sur filtre des cellules vivantes d'intérêt fixées, ou vivantes dans des conditions parfaitement compatibles avec leur mise en culture pour poursuivre des examens de caractérisation cytologique, et de cytogénétique, pour tout autre examen cellulaire ou pour extraire leur matériel génétique.

Cette récupération est effectuée directement sur filtre et pratiquement sans perte de cellules recherchées. On recueille ainsi, à moindre coût, une grande proportion des cellules considérées, en bon état, dans des conditions compatibles avec une culture de routine en laboratoire. Les cellules isolées peuvent être utilisées avant ou après culture, pour les examens de biologie cellulaire ou moléculaire.

Le dispositif objet de la présente invention permet aussi de recueillir du matériel cellulaire de cellules particulières, rapidement et efficacement, par exemple, après réalisation :
- d'une étape de filtration au cours de laquelle le principal du liquide et des dites autres cellules passe à travers un filtre dont les micropores ont un diamètre intermédiaire entre celui des dites cellules particulières et celui d'autres cellules,
- d'une étape de lyse suivie ou non d'amplification de l'ADN et/ou de l'ARN dans ledit compartiment et
- d'une étape de récupération du matériel génétique des cellules lysées, sur le filtre.

Le dispositif objet de la présente invention présente de nombreux avantages :
1/ la filtration peut être effectuée sur un portoir ou en tenant le dispositif à la main, ce qui représente un gain de temps et une économie de matériel importants (on évite une pompe à vide et un boîtier d'adaptation, notamment),
2/ la filtration peut être effectuée dans une hotte stérile.
3/ la filtration, peut être effectuée avec le dispositif en position oblique, voire pratiquement horizontal.
4/ les conditions sont parfaitement standardisées, le tube de prélèvement étant produit de façon standardisée avec une capacité de vide prédéfinie.
5/ les conditions d'utilisation présentent des conditions de sécurité élevées, l'opération pouvant être entièrement réalisée dans une hotte stérile ; de plus, le tube à vide, une fois rempli, peut être éliminé comme un tube habituel de prélèvement sanguin, le sang récupéré n'étant pas en contact avec l'opérateur.

Le dispositif objet de la présente invention, tel que succinctement exposé ci-dessus, comporte un moyen de liaison entre le compartiment et un cylindre de protection entourant l'aiguille et destiné à entourer, au moins partiellement, le tube à vide pendant l'aspiration du liquide à travers le filtre.

Grâce à ces dispositions, l'utilisateur est protégé contre une piqure accidentelle avec l'aiguille. De plus, la mise en place du tube à vide est facilitée par le guidage offert par le cylindre de protection. La position du tube à vide au cours de l'aspiration est aussi plus rigidement maintenue, ce qui évite son retrait ou un déplacement qui permettrait à de l'air d'entrer dans le tube à vide en passant à côté de l'aiguille dans le bouchon du tube à vide. Le risque de contamination de l'échantillon par l'utilisateur est minimisé car l'utilisateur ne peut toucher l'aiguille par mégarde.

Selon des caractéristiques particulières, le dispositif objet de la présente invention, tel que succinctement exposé ci-dessus, comporte, au moins provisoirement, ledit cylindre de protection.

Ainsi, la protection de l'aiguille, de l'échantillon et de l'utilisateur est assurée.

Selon des caractéristiques particulières, ledit moyen de liaison est provisoire et permet le retrait du cylindre de protection conjointement à celui du tube à vide et de l'aiguille.

Ainsi, la protection de l'aiguille et de l'utilisateur est assurée après la séparation du cylindre de protection du compartiment.

Selon des caractéristiques particulières, ledit cylindre de protection comporte une pellicule amovible obturant son ouverture opposée au moyen de liaison, ouverture par laquelle le tube à vide est introduit dans le cylindre de protection.

Grâce à ces dispositions, avant d'insérer le tube à vide, l'utilisateur retire la pellicule. Cette pellicule assure, à la fois, une meilleure protection, de l'utilisateur et de l'aiguille et une réduction des risqués de contamination de l'échantillon.

Selon des caractéristiques particulières, le dispositif objet de la présente invention comporte, en outre, un support de filtre amovible en acier chirurgical adapté à être solidarisé de manière temporaire à l'ouverture inférieure du compartiment.

Grâce à ces dispositions, on peut extraire le filtre avec son support pour cultiver ou analyser les cellules recueillies sur le filtre ou en extraire le matériel génétique. De plus, l'acier n'est pas toxique pour les cellules recueillies sur le filtre.

Selon des caractéristiques particulières, l'épaisseur dudit anneau est adaptée à permettre son passage dans un scanner.

Selon des caractéristiques particulières, ledit anneau porte un identifiant.

Ainsi, on peut associer cet identifiant, et donc les cellules recueillies, à un patient. On évite ainsi des erreurs.

L'ensemble du module de filtration étant stérile et préparé dans des normes habituellement appliquées pour la biologie moléculaire, on peut éviter toute contamination ou détérioration des cellules vivantes sur le filtre ou du matériel génétique. Le contenu du compartiment est maintenu à l'état stérile lors des manipulations sous une hotte à flux laminaire adaptée. Ainsi, toutes les étapes pour isoler et cultiver les cellules ou pour extraire et analyser leur matériel génétique peuvent être effectuées dans des conditions stériles.

Selon des caractéristiques particulières, le dispositif objet de la présente invention comporte un embout amovible fixé au compartiment de façon étanche et amovible et adapté à interdire le mouvement relatif du moyen mobile et du compartiment qui permet d'appliquer ladite force et libérer ledit support de filtre.

Grâce à ces dispositions, on garantit le maintien en position du porte-filtre. De plus, l'embout peut le protéger contre les éclaboussures et la contamination,

Ainsi, on retient le filtre pendant la filtration puis on le libère, pour sa récupération, en retirant l'embout et en mettant en mouvement respectif le moyen mobile et le compartiment pour appliquer la force qui libère le support de filtre.

Selon un deuxième aspect, la présente invention vise un procédé pour isoler des cellules vivantes sur un filtre ou extraire leur matériel génétique, caractérisé en ce qu'il comporte :
- une étape de solidarisation, au moins temporaire, d'un filtre à une ouverture inférieure d'un compartiment présentant, en outre, une entrée d'air,
- une étape d'insertion d'un liquide comportant lesdites cellules dans ledit compartiment,
- une étape de solidarisation, au moins temporaire d'une aiguille à ladite ouverture du compartiment, de manière étanche, le filtre se trouvant entre l'aiguille et le volume intérieur du compartiment,
- une étape de perforation d'un bouchon de tube à vide présentant une dépression par rapport à la pression ambiante avec ladite aiguille et
- une étape d'aspiration, par le biais de la dépression du tube à vide, du liquide à travers ledit filtre, ledit filtre retenant lesdites cellules et
- une étape de mise en mouvement relatif d'un moyen mobile présentant des pattes et du compartiment pour appliquer une force sur le filtre et libérer le filtre après aspiration du liquide à travers le filtre, les pattes du moyen mobile exerçant, lorsque le doigt d'un opérateur met en mouvement relatif le moyen mobile et le compartiment, une pression sur un support du filtre et libérant le support de filtre de l'ouverture du compartiment

Selon des caractéristiques particulières, le procédé objet de la présente invention, tel que succinctement exposé ci-dessus comporte, en outre :
- une étape de fixation, au moins temporaire, d'un cylindre de protection au compartiment, ledit cylindre de protection entourant alors l'aiguille et
- au cours de l'étape de perforation, on insère le tube à vide dans le cylindre de protection.

Les avantages, buts et caractéristiques particulières de ce procédé étant similaires à ceux du dispositif objet de la présente invention, tel que succinctement exposé ci-dessus, ils ne sont pas rappelés ici.

D'autres avantages, buts et caractéristiques de la présente invention ressortiront de la description qui va suivre, faite, dans un but explicatif et nullement limitatif, en regard des dessins annexés, dans lesquels :
- la figure 1 représente, schématiquement et en perspective, l'assemblage des pièces d'un premier mode de réalisation du dispositif objet de la présente invention,
- les figures 2A à 2D représentent, schématiquement et en sections axiales perpendiculaires entre elles, le premier mode de réalisation du dispositif avant utilisation,
- les figures 3A à 3O représentent, schématiquement, en élévation ou en coupe, des étapes de mise en oeuvre du premier mode de réalisation du dispositif objet de la présente invention,
- la figure 4 représente, sous forme d'un logigramme, des étapes mises en oeuvre dans un premier mode de réalisation particulier du procédé objet de la présente invention,
- la figure 5 représente, schématiquement et en perspective, l'assemblage des pièces d'un deuxième mode de réalisation particulier du dispositif objet de la présente invention,
- les figures 6A à 6D représentent, schématiquement et en sections axiales perpendiculaires entre elles, le deuxième mode de réalisation du dispositif avant utilisation,
- les figures 7A à 7L représentent, schématiquement, en élévation ou en coupe, des étapes de mise en oeuvre du deuxième mode de réalisation du dispositif objet de la présente invention,
- la figure 8 représente, en coupe, un support de filtre intégré au deuxième mode de réalisation du dispositif objet de la présente invention,
- la figure 9 représente, sous forme d'un logigramme, des étapes mises en oeuvre dans un deuxième mode de réalisation particulier du procédé objet de la présente invention,
- la figure 10 représente, schématiquement, un mode de réalisation particulier du dispositif, avant utilisation,
- la figure 11 représente, schématiquement, le mode de réalisation du dispositif illustré en figure 10, après retrait d'une pellicule de protection et avant insertion d'un tube à vide,
- la figure 12 représente, schématiquement, le mode de réalisation du dispositif illustré en figures 10 et 11, après insertion du tube à vide,
- la figure 13 représente, schématiquement, le mode de réalisation du dispositif illustré en figures 10 à 12, en cours de retrait du tube à vide et d'un cylindre de protection et
- la figure 14 représente, sous forme d'un logigramme, des étapes mises en oeuvre dans un mode de réalisation particulier du procédé objet de la présente invention.

Dans la description des figures qui va suivre, on envisage un système de filtration de liquide, notamment de sang, comportant des moyens de retrait d'un support de filtre. Cependant, la présente invention ne se limite pas à ces modes de réalisation préférentiels mais s'étend, au contraire, à tout système comportant un compartiment pour recevoir un liquide, un filtre monté, au moins temporairement, sur une ouverture de ce compartiment et une aiguille montée, au moins temporairement, de manière étanche, sur cette ouverture du compartiment pour que, lors du percement d'un bouchon de tube à vide pré-conditionné, l'aiguille véhicule une aspiration du liquide à travers le filtre et que des cellules d'intérêt soient retenues sur ce filtre.

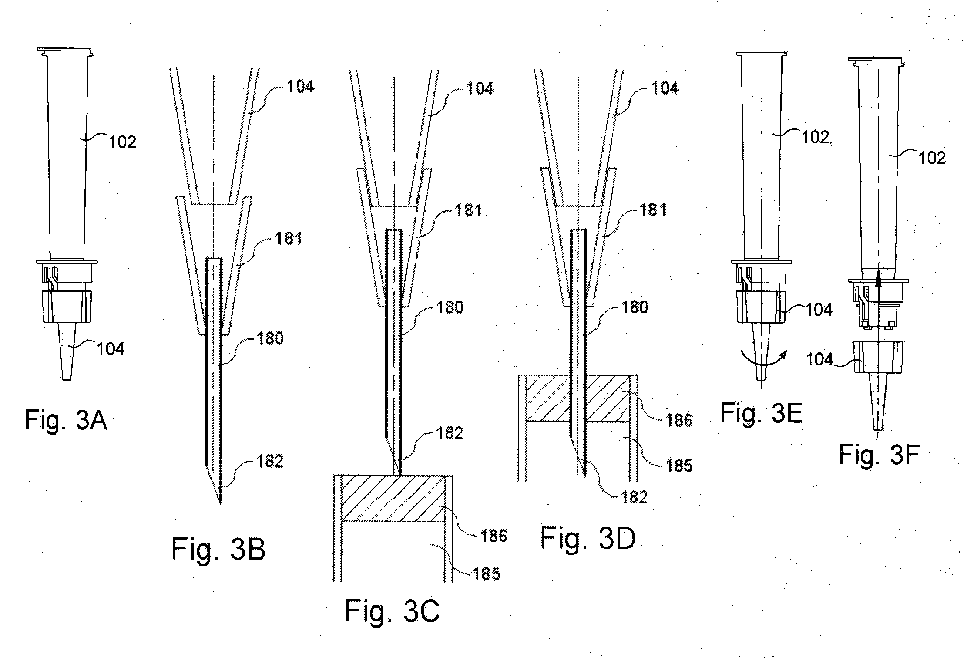

L'aiguille comporte une extrémité très fine adaptée à pénétrer dans le bouchon du tube à vide (voir figures 3B à 3D) et une embouchure, plus large adaptée à recevoir l'extrémité inférieure du compartiment ou d'un support de filtre. Le tube à vide présente une dépression très importante et un volume supérieur au volume de liquide à filtrer.

Dans des modes de réalisation préférentiels dans lesquels l'aiguille est amovible par rapport au compartiment, une fois le compartiment rempli avec le liquide comportant les cellules d'intérêt, pour récupérer les cellules, on emboîte l'embouchure de l'aiguille sur l'ouverture du compartiment puis on perfore le bouchon du tube à vide avec l'extrémité fine de l'aiguille. Sous l'effet de la dépression, la filtration du liquide s'effectue automatiquement, typiquement en 60 secondes.

Ainsi, la filtration peut être effectuée en tenant le dispositif à la main, ce qui représente un gain de temps et une économie de matériel importants, notamment parce qu'il n'est plus nécessaire de prévoir de pompe à vide ni de boîtier d'adaptation du compartiment sur la pompe.

De plus, la filtration peut être effectuée dans une hotte stérile, le dispositif pouvant être penché, voire pratiquement horizontal.

On va maintenant décrire des modes de réalisation avec un support de filtre amovible et des moyens de l'extraire du compartiment sans le toucher.

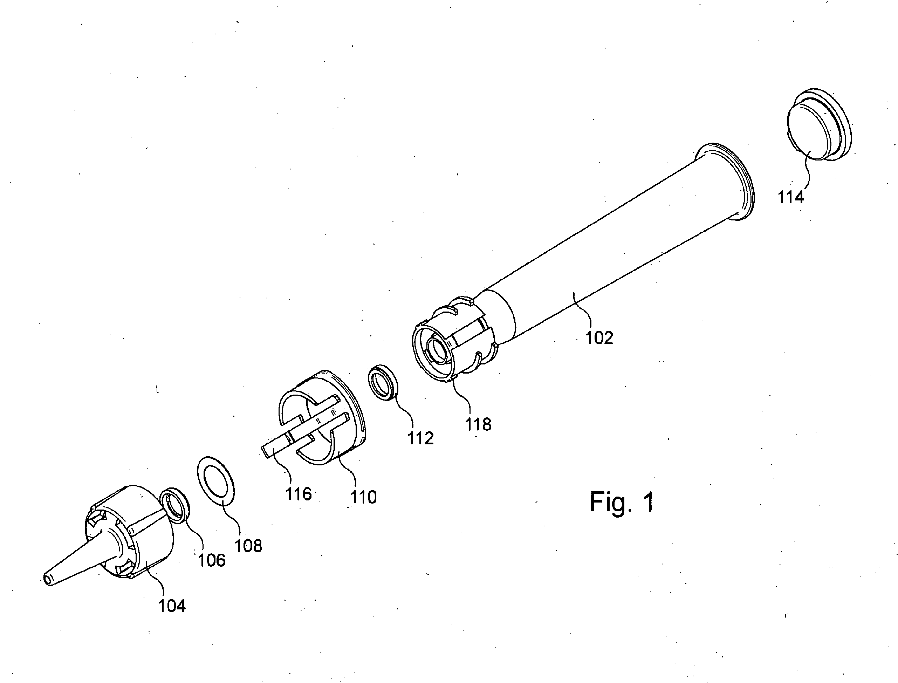

On observe, en figure 1, un réservoir, ou compartiment, 102, un embout 104, un joint 106, un filtre avec son support 108, un moyen mobile 110, un joint 112 et un bouchon 114 présentant une entrée d'air (non représentée).

Le compartiment 102 est de forme générale cylindrique. Son extrémité supérieure peut être obturée, de manière étanche, hormis l'entrée d'air, par le bouchon 114. L'extrémité inférieure du compartiment 102 présente, sur sa face externe, des anneaux discontinus dont les discontinuités guident des pattes du moyen mobile 110, lesdits anneaux guidant le corps du moyen mobile 110.

Ce moyen mobile 110 présente une forme générale cylindrique munie de deux pattes 116 s'étendant vers l'embout 104 et se resserrant dans cette direction de manière à être séparées, entre elles, d'une distance inférieure au diamètre du support de filtre 108. Comme on le verra, par la suite, cette forme particulière, notamment, celle des pattes 116 recourbées l'une vers l'autre, permet au moyen mobile 110, après retrait de l'embout 104, de pousser le support de filtre 108 afin de le libérer, ainsi que son filtre, du compartiment 102, lors du mouvement du moyen mobile vers le support de filtre 108.

L'extrémité des pattes 116 du moyen mobile 110 et l'extrémité inférieure du compartiment 102 sont adaptées à pénétrer dans une boîte ou puits, de culture. En revanche, l'anneau discontinu extrême du compartiment 102 possède un diamètre adapté à ce qu'il prenne appui sur le bord de la boîte, ou puits, de culture.

A l'ouverture inférieure du compartiment 102 est placé, de façon étanche, stérile et amovible, un embout, ou adaptateur, 104 qui enserre la paroi extérieure du compartiment 102 et présente une ouverture inférieure effilée de plus petit diamètre que celui du compartiment 102.

Cette ouverture inférieure effilée de l'embout, ou adaptateur, 104 présente une longueur suffisante pour permettre l'emboîtement mécanique étanche de l'embouchure d'une aiguille (voir figures 3B à 3D).

L'embout 104 présente, de manière coordonnée avec la forme de l'extrémité inférieure du compartiment 102, qui possède des ergots latéraux 118, des moyens de blocage par rotation, pour enserrer lesdits ergots, de manière connue en soi. L'embout 104 garantit ainsi le maintien en position du porte-filtre pendant les étapes de filtration. De plus, l'embout 104 protège le filtre contre les éclaboussures et la contamination.

L'extrémité inférieure du compartiment 102 présente une ouverture débouchant, après montage, sur le filtre porté par le support de filtre 108, lui-même retenu en position, d'une part, par l'extrémité inférieure du compartiment 102 et, d'autre part, par l'embout 104.

Le support de filtre 108 prend, dans le premier mode de réalisation, la forme d'une rondelle de forme annulaire. Le filtre est micro perforé et soudé sous le support de filtre 108 puis inséré avec elle dans l'extrémité inférieure du compartiment 102.

Préférentiellement, le support de filtre 108 amovible est annulaire et en acier chirurgical. L'acier n'est, en effet, pas toxique pour les cellules recueillies sur le filtre. De plus, un support de filtre en acier chirurgical est plus facile à retirer que s'il est en matière plastique et plus rigide. Préférentiellement, l'épaisseur dudit anneau est adaptée à permettre son passage dans un scanner. Préférentiellement, cet anneau porte un identifiant. Ainsi, on peut associer cet identifiant, et donc les cellules recueillies, à un patient. On évite ainsi des erreurs.

En variante, le support de filtre 108 est en PVC et possède une épaisseur inférieure ou égale à 0,4 mm, et préférentiellement, inférieure à 0,3 mm. Son diamètre extérieur est, par exemple, de 12,6 mm. Le diamètre du filtre porté par le support de filtre 108 est, par exemple, de 5,9 mm.

Le compartiment 102, l'embout 104 et le moyen mobile 110 sont réalisés, par exemple, en polypropylène. Les joints 106 et 112 sont, par exemple, en silicone.

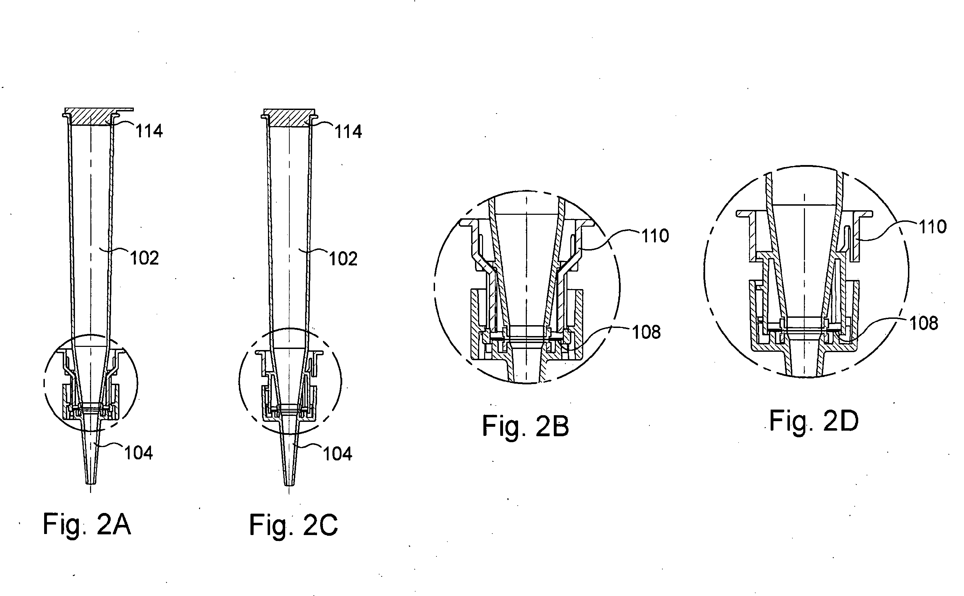

Les figures 2A et 2C sont des sections axiales, perpendiculaires entre elles, du premier mode de réalisation du dispositif objet de la présente invention, une fois assemblées les pièces illustrées en figure 1. Les figures 2B et 2D sont des vues de détails agrandies de parties des figures 2A et 2C, respectivement. On retrouve, dans les figures 2A à 2D, les éléments décrits en regard de la figure 1.

La figure 3A représente le dispositif en élévation, dans sa configuration de stockage. La figure 3B représente l'embout 104 lors de son insertion dans l'embouchure 181 d'une aiguille 180 qui présente une autre extrémité 182 très fine et biseauté pour faciliter le percement d'un bouchon de tube à vide. L'embouchure 181 de l'aiguille 180 est, préférentiellement, constituée en matière plastique. L'extrémité 182 de l'aiguille 180 est, préférentiellement métallique. La mise en place de l'aiguille 180 sur l'embout 104 peut se faire soit avant, soit après introduction de liquide (non représenté), par exemple du sang, dans le compartiment 102, à travers son ouverture supérieure.

La figure 3C illustre le début du percement du bouchon 186 du tube à vide 185, une fois l'aiguille 180 solidarisée de manière étanche à l'embout 104.

La figure 3D illustre le percement complet du bouchon 186 par l'aiguille 180, mettant en communication, à travers le filtre 108, l'intérieur du tube à vide 185, en dépression, et le volume du compartiment 102 comportant le liquide comportant les cellules d'intérêt. Le volume intérieur du tube à vide 185 est supérieur au volume de liquide à filtrer.

Lors de l'aspiration, certaines cellules particulières du liquide présent dans le compartiment 102, de plus fort diamètre, sont retenues par le filtre 108 alors que le principal du liquide, du contenu et, le cas échéant, de la paroi des cellules lysées et lés cellules de plus petites dimensions que les cellules à recueillir sont aspirés dans le tube à vide 185, à travers le filtre 108.

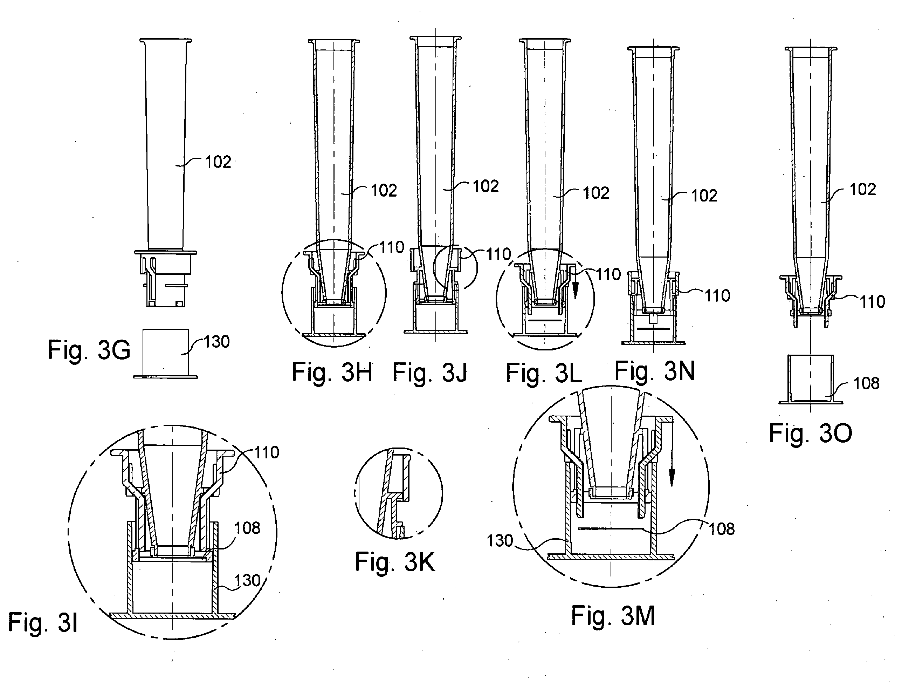

Puis, comme illustré en figures 3E et 3F, on retire l'embout 104 après une rotation le libérant des ergots 118. Puis, comme illustré en figures 3G et 3H, on insère l'extrémité du compartiment 102 dans une boîte, ou un puits, de culture 130.

Comme exposé ci-dessus et comme illustré en figure 3I, l'extrémité rapprochée des pattes 116 du moyen mobile 110 et l'extrémité inférieure du compartiment 102 sont adaptés à pénétrer dans une boîte ou puits, de culture. En revanche, l'anneau discontinu extrême du compartiment 102 possède un diamètre adapté à ce qu'il prenne appui sur le bord de la boîte, ou puits, de culture 130.

Plus précisément, comme illustré en figures 3J et 3K, le moyen mobile 110 peut encore, dans cette position, bouger parallèlement à l'axe du compartiment 102.

Comme illustré en figures 3N et 3M, lors de ce mouvement, les pattes 116 du moyen mobile mis en mouvement par les doigts d'un opérateur, exercent une force verticale, de haut en bas, sur le support de filtre 108 et le libère de l'extrémité inférieure du compartiment 102. Le filtre et son support 108 tombent alors dans la boîte, ou puits, de culture 130.

Enfin, comme illustré en figure 3O, on retire le compartiment 102 et le moyen mobile 110 de la boîte, ou puits, de culture 130.

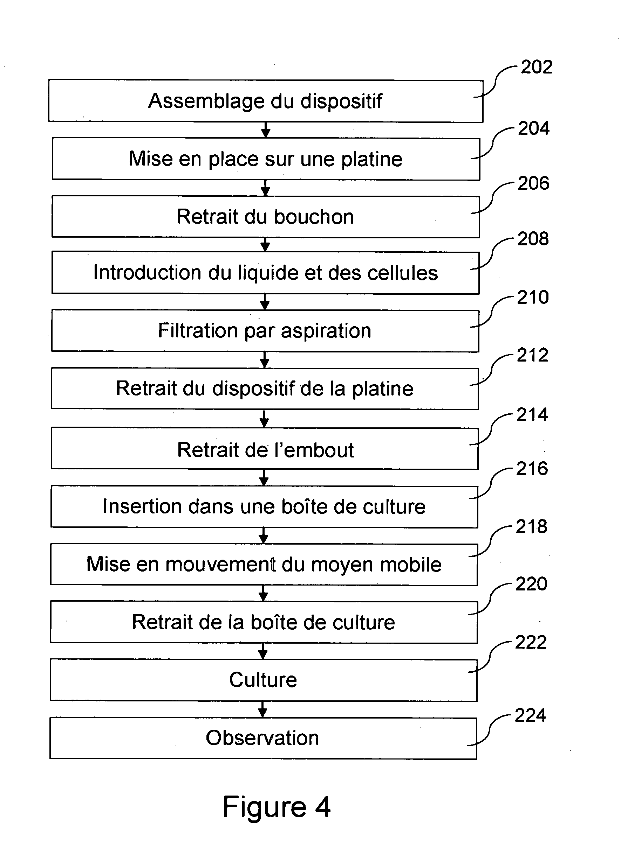

La figure 4 récapitule les étapes ainsi mises en oeuvre.

Au cours d'une étape 202, on assemble les pièces du dispositif. Au cours d'une étape 203, on retire le bouchon 114. Au cours d'une étape 204, on insert l'embout 104 dans l'embouchure 181 de l'aiguille 180. Au cours d'une étape 205, on introduit un liquide, par exemple, du sang, contenant des cellules à isoler et éventuellement à cultiver, par l'extrémité supérieure du compartiment 102.

Au cours d'une étape 206, on applique l'extrémité pointue 182 de l'aiguille 180 approximativement au centre du bouchon 186 et on applique, sur le compartiment 102, une pression pour faire pénétrer l'aiguille dans le bouchon 186 jusqu'à ce que l'extrémité de l'aiguille atteigne le volume intérieur, en dépression, voire vide, du tube à vide 185.

Au cours d'une étape 210, on effectue une filtration, par aspiration dans le tube à vide 185, des cellules plus petites que les cellules d'intérêt, les éventuelles cellules lysées, et le principal du liquide présent dans le compartiment 102, les cellules d'intérêt restant retenues sur le filtre 108.

Au cours d'une étape 212, on retire le tube à vide 185 et l'aiguille 180. Au cours d'une étape 214, on retire l'embout 104. Au cours d'une étape 216, on insère l'extrémité du compartiment 102 dans une boîte, ou un puits, de culture.

Au cours d'une étape 218, on met en mouvement respectif le moyen mobile et le compartiment pour exercer une force sur le support de filtre 108 et le libérer afin qu'il tombe avec son filtre dans la boîte, ou puits, de culture 130. Au cours d'une étape 220, on retire le compartiment 102 et le moyen mobile 110 de la boîte, ou puits, de culture 130.

Au cours d'une étape 222, la culture est réalisée, de manière connue, dans le puits de culture 130. On observe que la présence du support 108 autour de la face supérieure du filtre, face qui porte les cellules isolées sur filtre, permet d'éviter que ces cellules ne quittent le filtre.

Lors de l'étape 222 de mise en culture des cellules vivantes d'intérêt sur le filtre, le filtre est, par exemple, recouvert d'une mince couche de Matrigel (ou mis en contact avec une couche de Matrigel, marque déposée, préalablement mis au fond du puits de plaque et/ou du flacon de culture et sur laquelle il repose) contenant des facteurs adaptés à la croissance des cellules d'intérêt.

Lorsque l'on souhaite observer les cellules, ou leur matériel génétique, au cours d'une étape 224, on attrape le support de filtre 108, dont la saisie avec une brucelle est facilitée par la présence de trous cylindriques latéraux, ou encoches, dans la face supérieure du support de filtre 108.

Puis, on peut poser le support de filtre 108 sur une lame de verre et on appliquer, sur le filtre, une lamelle de verre en forme de disque de diamètre adapté à la surface supérieure libre du filtre. L'analyse des cellules ou l'extraction de leur matériel génétique est effectuée de manière connue en soi.

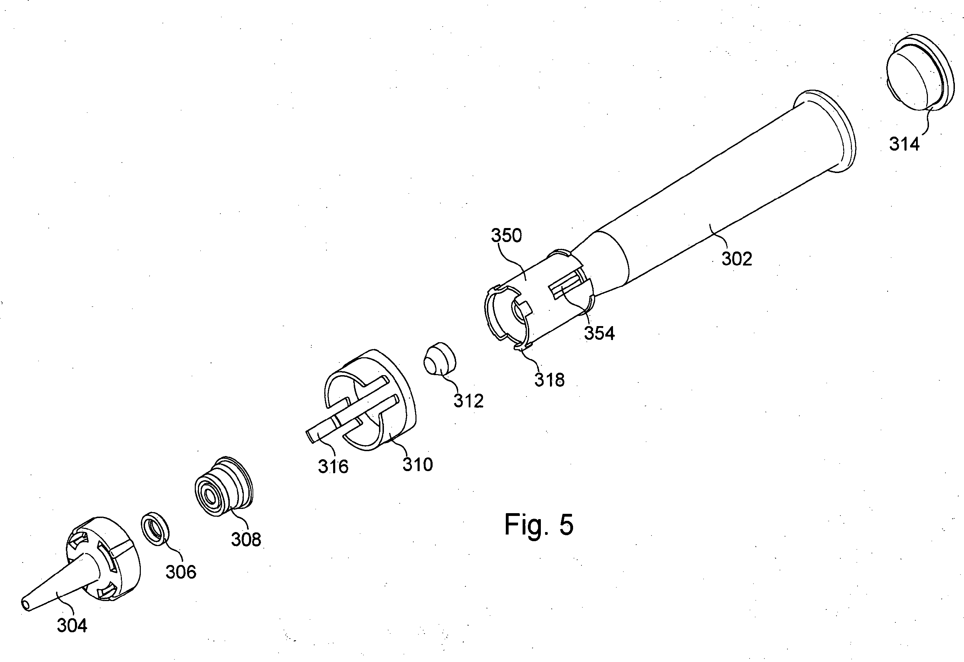

On observe, en figure 5, un réservoir, ou compartiment, 302, un embout 304, un joint 306, un support de filtre 308, un moyen mobile 310, un joint 312 et un bouchon 314.

Le compartiment 302 est de forme générale cylindrique. Son extrémité supérieure peut être obturée, de manière étanche, à l'exception d'une entrée d'air (non représentée), par le bouchon 314. L'extrémité inférieure du compartiment 302 présente, sur sa face externe, un cylindre 350 séparé du corps du compartiment 302, hormis par des liaisons mécaniques en forme de nervures latérales 352. Ce cylindre 350 possède un diamètre extérieur qui correspond au diamètre intérieur du corps du moyen mobile 310, afin de le guider en déplacement. Le cylindre 350 est muni de lumières 354 adaptées à laisser pénétrer et coulisser longitudinalement les pattes 316 du moyen mobile 310.

Le moyen mobile 310 présente une forme générale cylindrique munie de deux pattes 316 s'étendant vers l'embout 304 et se resserrant dans cette direction de manière à être séparées, entre elles, d'une distance inférieure au diamètre du support de filtre 308. Comme on le verra, par la suite, cette forme particulière, notamment, celle des pattes 316 recourbées l'une vers l'autre, permet au moyen mobile 310, après retrait de l'embout 304, de pousser le support de filtre 308 afin de le libérer du compartiment 302, lors du mouvement du moyen mobile vers le support de filtre 308.

L'embout 304 présente, de manière coordonnée avec la forme de l'extrémité inférieure du compartiment 302, qui possède des ergots latéraux 318, des moyens de blocage par rotation, pour enserrer lesdits ergots, de manière connue en soi. L'embout 304 garantit ainsi le maintien en position du porte-filtre pendant les étapes de filtration. De plus, l'embout 304 protège le filtre contre les éclaboussures et la contamination.

L'extrémité inférieure du compartiment 302 présente une ouverture débouchant, après montage, sur le filtre porté par le support de filtre 308, lui-même retenu en position, d'une part, par l'extrémité inférieure du compartiment 302 et, d'autre part, par l'embout 304.

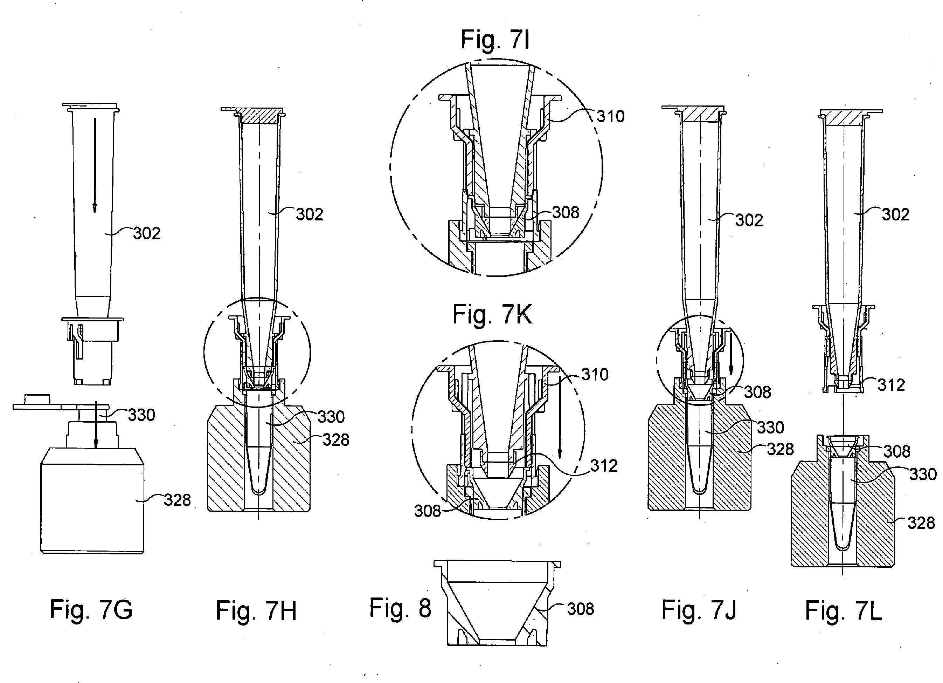

Comme illustré en figure 8, le support de filtre 308 prend, dans le deuxième mode de réalisation, une forme coordonnée à celle d'un tube Eppendorf.

En particulier :
- le support 308 présente, dans sa partie supérieure intérieure, la forme de la partie supérieure intérieure d'un tube Eppendorf, ce qui permet de fermer l'ouverture supérieure dudit support avec un bouchon de tube Eppendorf et
- le support 308 présente, dans sa partie supérieure extérieure simule la forme de la partie supérieure intérieure d'un tube Eppendorf ce qui permet au support de filtre 308 de s'insérer dans la partie haute d'un tube Eppendorf.

De plus, le support de filtre 308 présente une fonction colonne pour permettre la lyse des cellules retenues sur filtre et le transfert par centrifugation du lysat cellulaire et du matériel génétique du support de filtre vers le tube Eppendorf.

Le support de filtre 308 est, préférentiellement, en polycarbonate. Le compartiment 302, l'embout 304 et le moyen mobile 310 sont réalisés, par exemple, en polypropylène. Les joints 306 et 312 sont, par exemple, en silicone.

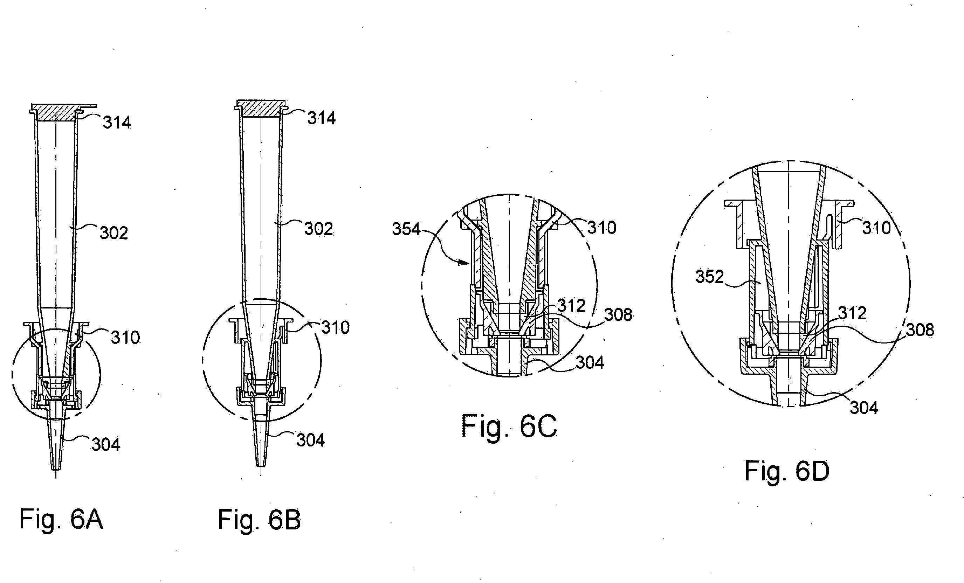

Les figures 6A et 6C sont des sections axiales, perpendiculaires entre elles, du deuxième mode de réalisation du dispositif objet de la présente invention, une fois assemblées les pièces illustrées en figure 5. Les figures 6B et 6D sont des vues de détails agrandies de parties des figures 6A et 6C, respectivement. On retrouve, dans les figures 6A à 6D, les éléments décrits en regard de la figure 5.

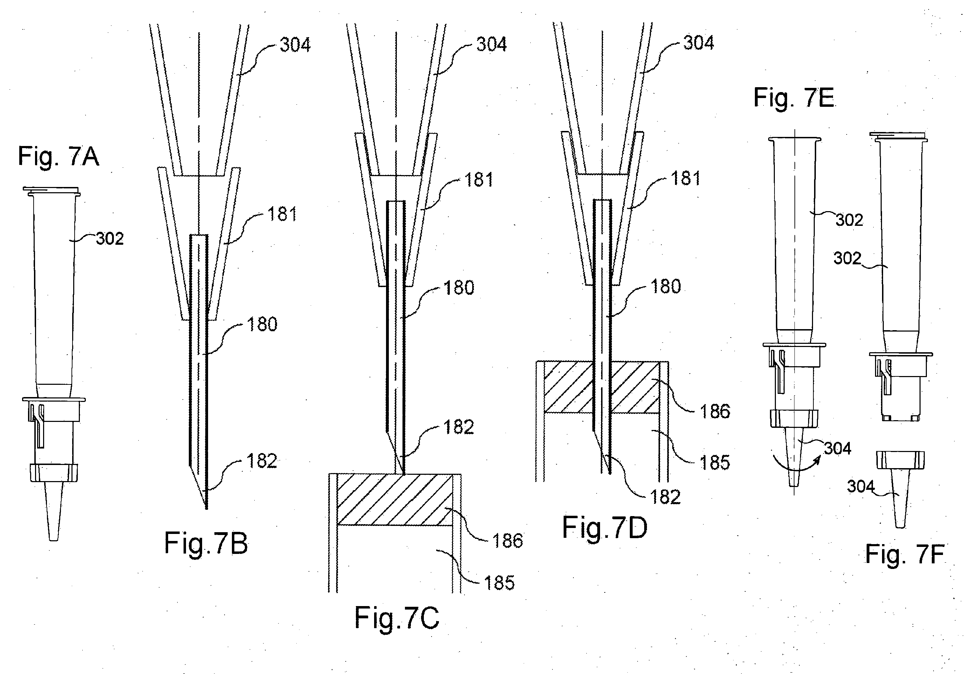

La figure 7A représente le dispositif en élévation, dans sa configuration de stockage. Les figures 7B à 7D sont identiques aux figures 3B à 3D, à ceci près que l'embout est référencé 304.

Comme illustré en figures 7E et 7F, on retire l'embout 304 après une rotation le libérant des ergots 318. Puis, comme illustré en figures 7G, 7H et 7I, on insère l'extrémité du compartiment 302 dans un support de tube Eppendorf 328 muni d'un tube Eppendorf 330.

Comme illustré en figures 7J et 7K, le moyen mobile 310 est ensuite mis en mouvement vers le bas, parallèlement à l'axe du compartiment 302. Lors de ce mouvement, les pattes 316 du moyen mobile 310 mis en mouvement par les doigts d'un opérateur, exercent une force verticale, de haut en bas, sur le support de filtre 308 et le libère de l'extrémité inférieure du compartiment 302. Le support de filtre 308 s'enfonce alors dans le tube Eppendorf 330.

Enfin, comme illustré en figure 7L, on retire le compartiment 302 et le moyen mobile 310.

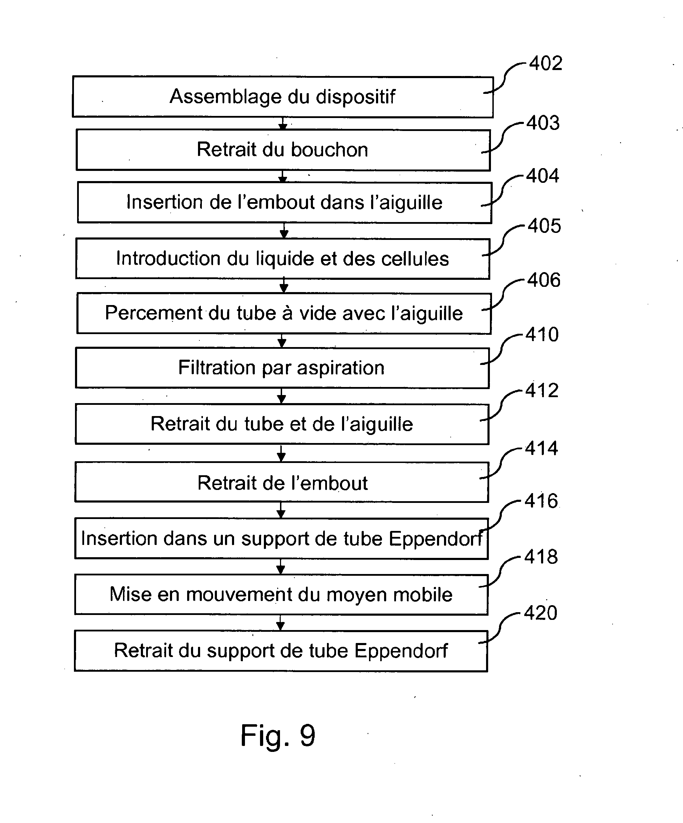

La figure 9 récapitule les étapes ainsi mises en oeuvre.

Au cours d'une étape 402, on assemble les pièces du dispositif. Au cours d'une étape 403, on retire le bouchon 314. Au cours d'une étape 404, on insert l'embout 304 dans l'embouchure 181 de l'aiguille 180. Au cours d'une étape 405, on introduit un liquide, par exemple, du sang, contenant des cellules à filtrer et éventuellement cultiver, par l'extrémité supérieure du compartiment 302.

Au cours d'une étape 406, on applique l'extrémité pointue 182 de l'aiguille 180 approximativement au centre du bouchon 186 et on applique, sur le compartiment 302, une pression pour faire pénétrer l'aiguille dans le bouchon 186 jusqu'à ce que l'extrémité de l'aiguille atteigne le volume intérieur, en dépression, voire vide, du tube à vide 185.

Au cours d'une étape 410, on effectue une filtration, par aspiration dans le tube à vide 185, des cellules plus petites que les cellules d'intérêt et les éventuelles cellules lysées et le principal du liquide présent dans le compartiment 302.

Au cours d'une étape 412, on retire le tube à vide 185 et l'aiguille 180. Au cours d'une étape 414, on retire l'embout 304. Au cours d'une étape 416, on insère l'extrémité du compartiment 302 dans un support de tube Eppendorf.

Au cours d'une étape 418, on met en mouvement respectif le moyen mobile et le compartiment pour exercer une force sur le support de filtre et le libérer afin qu'il s'enfonce dans le tube Eppendorf. Enfin, au cours d'une étape 420, on retire le compartiment 302 et le moyen mobile 310 et on referme le bouchon du tube Eppendorf.

La mise en oeuvre du tube Eppendorf est ensuite réalisée de manière connue en soi, par exemple avec des étapes de lyse, de centrifugation et de récupération du matériel génétique avec ou sans pré-amplification du génome global.

Au cours d'étapes 422 et 424, on effectue une analyse du matériel génétique, notamment l'ADN ou l'ARN des cellules recherchées, recueilli au fond d'un tube Eppendorf après centrifugation. L'ADN amplifié est utilisé comme matrice pour détecter les mutations de sensibilité ou de résistance aux thérapies ciblées. De plus, ou alternativement, l'ADNc (en anglais « cDNA », « c » signifiant « complémentaire ») provenant de l'ARN par conversion par RT et amplifié est utilisé comme matrice pour détecter le niveau d'expression de gènes de sensibilité ou de résistance aux thérapies ciblées. Ce matériel génétique, lorsqu'il est obtenu de trophoblastes filtrés à partir du sang de femmes enceintes, peut être utilisé pour permettre l'identification d'anomalies génétiques éventuelles.

Un volume défini du matériel génétique amplifié, notamment l'ADN, est prélevé pour détecter les mutations de sensibilité ou de résistance aux thérapies ciblées à l'aide de couples d'amorces (en anglais « primers ») sens et anti-sens et de couples de sondes et au cours d'une PCR (acronyme de « polymerase chain reaction ») quantitative et en temps réel.

Le principe de la recherche de mutations de sensibilité ou de résistance aux thérapies ciblées mis en oeuvre dans le mode de réalisation représenté est le suivant. La discrimination allélique ou « SNP genotyping assay » (SNP étant l'acronyme de « single nucleotide polymorphism » pour polymorphisme nucléotide simple) permet de renseigner sur la présence ou l'absence d'une mutation ponctuelle au niveau d'un gène. La première étape, 422, d'une discrimination allélique est une réaction de PCR quantitative en temps réel réalisée avec deux amorces pour amplifier la séquence d'intérêt et deux sondes, par exemple de type TaqMan (marque déposée). L'une des sondes reconnaît la séquence mutée et l'autre reconnaît la séquence normale. Les deux sondes sont associées à des fluorochromes différents, par exemple « VIC » pour la sonde s'hybridant à la séquence normale et « FAM » pour la sonde s'hybridant à la séquence mutée. La seconde étape, 424, fait appel à un programme de discrimination allélique mesurant la fluorescence initiale et la fluorescence finale émise par les fluorochromes FAM ou/et VIC. Ce programme permet de faire la distinction entre les différentes séquences présentes dans chaque échantillon :
- une augmentation de la fluorescence uniquement en VIC indique un profil homozygote pour la séquence normale,
- une augmentation de la fluorescence uniquement en FAM indique un profil homozygote pour la séquence mutée,
- une augmentation de la fluorescence à la fois en VIC et en FAM indique un profil hétérozygote.

Les sondes s'apparient entre les deux amorces et révèlent la présence ou l'absence d'une mutation du fait de leur couleur de fluorescence associée.

Dans d'autres modes de réalisation, un volume défini du matériel génétique notamment l'ARN, converti en ADNc par RT (acronyme de « reverse transcription ») et amplifié, est prélevé pour détecter le niveau d'expression de gène de sensibilité ou de résistance aux thérapies ciblées à l'aide de couples d'amorces sens et anti-sens et d'une sonde et au cours d'une PCR (acronyme de « polymerase chain reaction ») quantitative et en temps réel, par exemple avec 50 cycles.

Dans chacun des modes de réalisation décrits ci-dessus, préférentiellement, le filtre est réalisé en polycarbonate avec un traitement de surface hydrophile. L'utilisation d'un tel filtre améliore le taux de retenu des cellules particulières et réduit l'adhérence des autres cellules ou de leur contenu, lorsqu'elles ont été spécifiquement lysées.

Le filtre présente, préférentiellement, un diamètre de pores centré sur une valeur inférieure, par exemple de 1 µm, à la valeur correspondante mise en oeuvre pour les mêmes cellules fixées, c'est-à-dire rendues rigides.

Par exemple, si, pour les cellules fixées, le diamètre des pores aurait été centré sur 7,5 µm, il est ici centré sur une valeur inférieure, par exemple de 6,5 µm. Du fait de la dispersion des diamètres, pratiquement aucun pore ne possède alors un diamètre supérieur à 7 µm.

Pour une application de l'invention à des cellules sanguines, le filtre présente des pores dont la densité est entre 50.000 et 200.000 pores/cm² et, préférentiellement d'environ 100.000 pores/cm².

Grâce à l'usage d'un filtre en polycarbonate, la dépression nécessaire est beaucoup plus limitée que dans les systèmes de l'art antérieur, jusqu'à être quatre fois moindre, ce qui évite de détériorer les cellules que l'on vise à recueillir sur le filtre.

Dans des variantes, le support 108 ou 308 de filtre est mécaniquement lié au moyen mobile jusqu'à l'application, par mouvement du compartiment vers le puits de culture ou le tube Eppendorf, de la force qui libère le support de filtre du moyen mobile. L'inversion des rôles de l'extrémité inférieure du compartiment 102 ou 302 et du moyen mobile 110 ou 310 pour que ce soit ce dernier qui tienne le support de filtre dans ou devant une lame de verre, un puits de culture ou un tube Eppendorf et le premier qui le libère lors d'un appui sur l'extrémité supérieure du compartiment est une adaptation aisée, à la portée de l'homme du métier à partir de la description des modes de réalisation donnée ci-dessus.

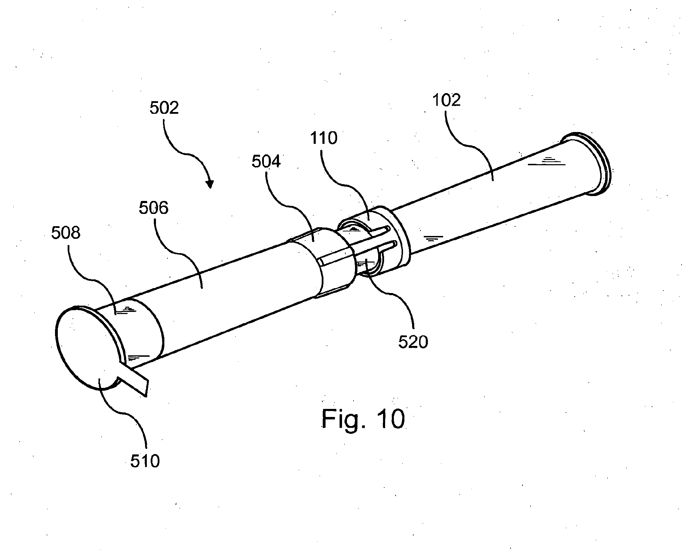

Les figures 10 à 14 concernent des modes de réalisation particuliers du dispositif et du procédé objets de la présente qui mettent en oeuvre un cylindre de protection et de guidage du tube à vide.

Bien que ces modes de réalisations particuliers complètent l'un ou l'autre des modes de réalisation décrits ci-dessus, on a choisi, pour réaliser les figures 10 à 14, de les adapter au mode de réalisation illustré aux figures 1 à 4.

On observe, en figures 10 à 13, le compartiment 102, le moyen mobile 110 et un cylindre de protection 502 lié au compartiment 102 par un moyen de liaison en deux parties, 504 et 520.

Le cylindre de protection 502 comporte la partie 504 du moyen de liaison, une partie dépolie 506, une partie transparente 508 et, sur une ouverture opposée au compartiment 102, une pellicule 510.

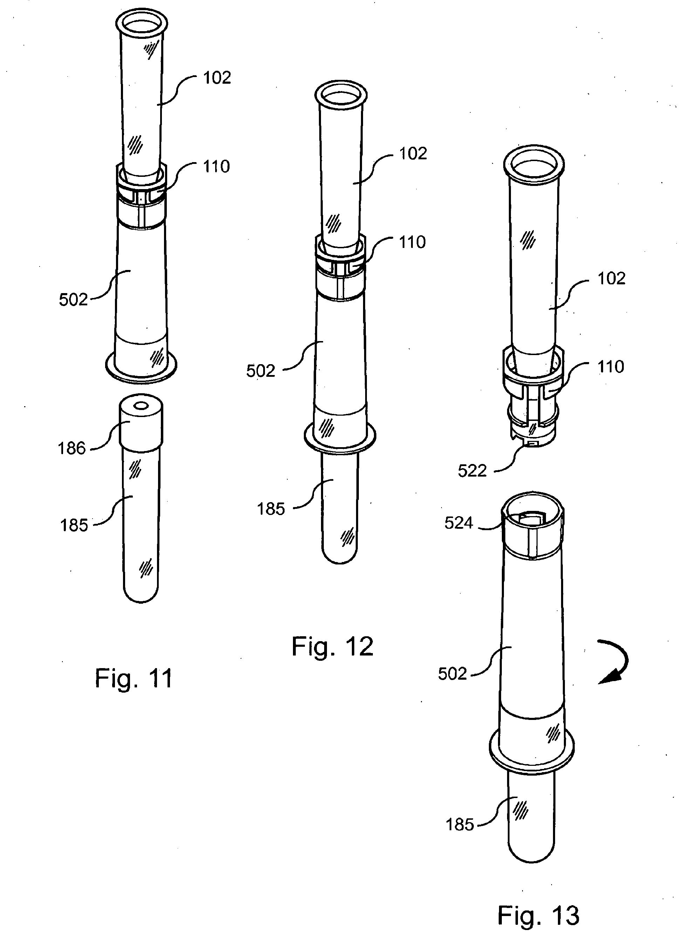

La partie 520 est formée dans l'extrémité du compartiment 102. La figure 13 montre un mode de réalisation particulier de la partie 520 constituée de quatre dents 522 formées latéralement sur une partie cylindrique co-axiale avec le compartiment 102. La partie 504 est, dans ce mode de réalisation, constituée de quatre gorges 524 dont les profils correspondent à celui des dents. Ces gorges 524 s'étendent, de manière héliptique, depuis une ouverture adaptée à recevoir une dent 522 vers l'intérieur du cylindre de protection 502 de telle manière que la rotation du cylindre de protection 502 indiquée par une flèche sur la figure 13 provoque l'avancée de chaque dent 522 dans la gorge 524 correspondante et le serrage du cylindre de protection 502 sur le compartiment 102.

La fixation du cylindre de protection 502 sur l'embout comportant l'aiguille 180 est la suivante. L'aiguille 180 est enchâssée dans la partie inférieure de la partie 524, qui est la partie opposée au compartiment 502. La partie 524 est montée en force dans la partie 506 grâce à quatre ailettes. Les ailettes se trouvent sur la partie 504 et sont insérées dans quatre gorges qui se trouvent sur la partie 506.

La partie dépolie 506 sert à masquer l'aiguille 180. La partie transparente 508 permet à l'utilisateur de vérifier l'état de la filtration et sa complétion.

La pellicule 510, qui recouvre et obture l'intégralité de l'ouverture inférieure du cylindre 502 est munie d'une partie latérale s'étendant à courte distance du cylindre 502 (illustrée en figure 10). Cette partie latérale permet le retrait aisé de la pellicule 510.

La pellicule 510 protège l'utilisateur contre l'accès à l'aiguille 180. La pellicule 510 protège aussi l'aiguille 180 contre les risques d'encrassement et/ou de contamination.

Le cylindre 502 est discrètement coloré, par exemple en bleu, vert ou jaune, selon la destination du dispositif de filtration (études cytologiques, biologie moléculaire, culture, respectivement).

On observe, en figure 11, qu'après insertion du liquide à filtrer dans le compartiment 102 et retrait de la pellicule 510, on insère le tube à vide 185 muni de son bouchon 186, dans le cylindre de protection et de guidage 502. On appuie ensuite sur le tube à vide 185 pour que l'aiguille 180 perce le bouchon 186, comme exposé plus haut.

Dans l'assemblage ainsi constitué, illustré en figure 12, la dépression initialement présente dans le tube à vide 185 provoque le filtrage du liquide présent dans le compartiment 102.

Une fois le filtrage terminé, comme illustré en figure 13, on retire conjointement le cylindre de protection 502 et l'aiguille 180 qu'il renferme.

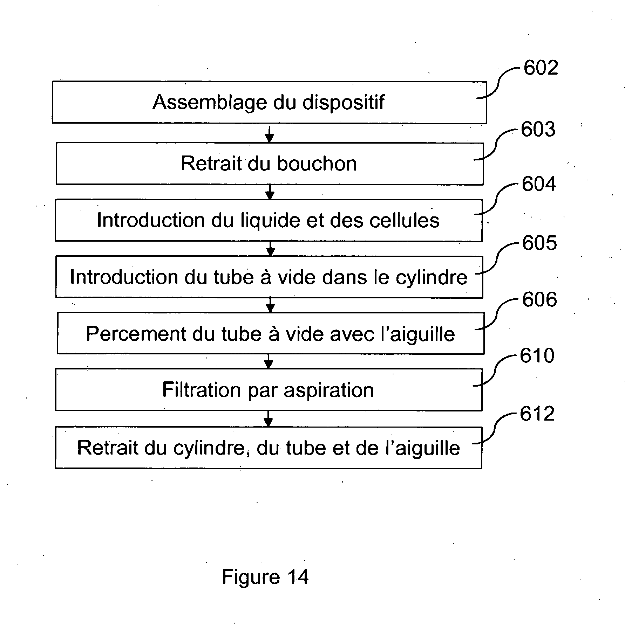

La figure 14 récapitule les étapes ainsi mises en oeuvre.

Au cours d'une étape 602, on assemble les pièces du dispositif. Au cours d'une étape 603, on retire le bouchon 114. Au cours d'une étape 604, on introduit un liquide, par exemple, du sang, contenant des cellules à isoler et éventuellement à cultiver, par l'extrémité supérieure du compartiment 102.

Au cours d'une étape 605, on introduit le tube à vide 185 dans le cylindre de protection 502, ce qui a pour effet d'appliquer l'extrémité pointue 182 de l'aiguille 180 approximativement au centre du bouchon 186, et on applique, sur le compartiment 102 et sur le tube à vide 185, des forces opposées pour faire pénétrer l'aiguille 180 dans le bouchon 186 jusqu'à ce que l'extrémité de l'aiguille atteigne le volume intérieur, en dépression, voire sous vide, du tube à vide 185, au cours d'une étape 606.

Au cours d'une étape 610, on effectue une filtration, par aspiration dans le tube à vide 185, des cellules plus petites que les cellules d'intérêt, les éventuelles cellules lysées, et le principal du liquide présent dans le compartiment 102, les cellules d'intérêt restant retenues sur le filtre 108.

Au cours d'une étape 612, on retire le cylindre de protection 502, tube à vide 185 et l'aiguille 180.

Les étapes suivantes ont déjà été exposées en regard des autres modes de réalisation et dépendent de ces modes de réalisation. Elles ne sont donc pas décrites, de nouveau, ici.

A la lecture de ce qui précède, on comprend que, tant que le cylindre de protection est lié au compartiment, l'utilisateur est protégé contre une piqure accidentelle avec l'aiguille. De plus, la mise en place du tube à vide est facilitée par le guidage offert par le cylindre de protection. La position du tube à vide au cours de l'aspiration est aussi plus rigidement maintenue, ce qui évite son retrait ou un déplacement qui permettrait à de l'air d'entrer dans le tube à vide en passant à côté de l'aiguille dans le bouchon du tube à vide. Le risque de contamination de l'échantillon par l'utilisateur est minimisé car l'utilisateur ne peut toucher l'aiguille par mégarde.

Préférentiellement, comme illustré aux figures 10 à 13, le dispositif objet de la présente invention comporte, au moins provisoirement, ledit cylindre de protection. Dans d'autres modes de réalisation, le montage est effectué juste avant utilisation.

Préférentiellement, comme illustré aux figures 10 à 13, le moyen de liaison est provisoire et permet le retrait du cylindre de protection conjointement à celui du tube à vide. Dans des variantes, le retrait de ces deux parties est séparé en deux étapes successives.

Dans des modes de réalisation, la présentation du dispositif objet de la présente invention prend la forme d'un kit comportant, dans un sachet externe, deux sachets internes dont :
- le premier comporte le dispositif monté, tel qu'illustré en figures 2A, 6A ou 10 et
- le second comporte l'aiguille, le tube à vide, le puits de culture et unie lame circulaire et/ou un tube Eppendorf ou tout autre accessoire utile à la mise en oeuvre du dispositif.

La mise en oeuvre de la présente invention permet d'éviter des prélèvements risqués de cellules, par exemple de cellules du liquide amniotiques tout en permettant des cultures, par exemple pour une amniocentèse. De plus, du fait de la forme en réservoir du support 108 du filtre, on peut réaliser directement dans ce support des réactions d'immunocytochimie ou d'hybridation fluorescente in situ (« FISH »).

## Revendications

1. Dispositif pour isoler des cellules fixées ou vivantes sur un filtre (108, 308) ou extraire le matériel génétique des dites cellules, qui comporte :
- un compartiment (102, 302) présentant un volume intérieur pour recevoir un liquide comportant lesdites cellules, une ouverture inférieure et une entrée d'air,
- un moyen (110) mobile par rapport au compartiment, ce moyen mobile présentant des pattes,
- le filtre solidaire, au moins temporairement, de ladite ouverture du compartiment ou audit moyen mobile, adapté à retenir lesdites cellules lors du passage du liquide à travers le filtre et
- une aiguille (180) solidaire, au moins temporairement, de ladite ouverture du compartiment, de manière étanche, le filtre se trouvant entre l'aiguille et le volume intérieur du compartiment, ladite aiguille étant adaptée à perforer un bouchon (186) de tube à vide (185) présentant une dépression par rapport à la pression ambiante pour aspirer le liquide à travers ledit filtre,
**caractérisé en ce qu'**il comporte, en outre un moyen de liaison (504, 520) entre le compartiment et un cylindre de protection (502) entourant l'aiguille, ledit moyen de liaison étant adapté à entourer, au moins partiellement, le tube à vide pendant l'aspiration du liquide à travers le filtre,
dans lequel le moyen mobile et le compartiment sont configurés pour être mis en mouvement relatif pour appliquer une force sur le filtre et libérer le filtre après aspiration du liquide à travers le filtre, et dans lequel les pattes du moyen mobile sont configurés pour, lorsque le doigt d'un opérateur met en mouvement relatif le moyen mobile et le compartiment, exercer une pression sur un support du filtre et libérer le support de filtre de l'ouverture du compartiment.

2. Dispositif selon la revendication 1, qui comporte, au moins provisoirement, ledit cylindre de protection (502).

3. Dispositif selon l'une quelconque des revendications 1 ou 2, dans lequel ledit moyen de liaison (504, 520) est provisoire et permet le retrait conjoint du cylindre de protection (502) et du tube à vide (185).

4. Dispositif selon l'une quelconque des revendications 1 à 3, dans lequel ledit cylindre de protection (502) comporte une pellicule amovible (510) obturant une ouverture du cylindre de protection opposée au moyen de liaison (504, 520), ouverture adaptée à l'introduction du tube à vide (185) dans le cylindre de protection après retrait de la pellicule amovible.

5. Dispositif selon l'une quelconque des revendications 1 à 4, dans lequel le support de filtre (108, 308) amovible est en acier chirurgical et est adapté à être solidarisé de manière temporaire à l'ouverture inférieure du compartiment (102, 302).

6. Dispositif selon la revendication 5, dans lequel l'épaisseur dudit support de filtre (108, 308) est adaptée à permettre son passage dans un scanner.

7. Dispositif selon l'une quelconque des revendications 5 ou 6, dans lequel ledit support de filtre (108, 308) porte un identifiant.

8. Dispositif selon l'une des revendications 1 à 7, qui comporte un embout amovible fixé au compartiment de façon étanche et amovible et adapté à interdire le mouvement relatif du moyen mobile et du compartiment qui permet d'appliquer ladite force et libérer ledit support de filtre.

9. Procédé pour isoler des cellules vivantes sur un filtre (108, 308) ou extraire le matériel génétique des dites cellules, qui comporte :
- une étape (202, 402, 602) de solidarisation, au moins temporaire, d'un filtre (108, 308) à une ouverture inférieure d'un compartiment (102, 302) présentant, en outre, une entrée d'air,
- une étape (208, 405, 604) d'insertion d'un liquide comportant lesdites cellules dans ledit compartiment et
- une étape de solidarisation (202, 402, 602), au moins temporaire, d'une aiguille (180) à ladite ouverture du compartiment, de manière étanche, le filtre se trouvant entre l'aiguille et le volume intérieur du compartiment,
**caractérisé en ce qu'**il comporte, en outre :
- une étape (602) de fixation, au moins temporaire, d'un cylindre de protection (502) au compartiment, ledit cylindre de protection entourant alors l'aiguille,
- une étape de perforation (210, 406, 605, 606), avec ladite aiguille, d'un bouchon (186) de tube à vide (185) présentant une dépression par rapport à la pression ambiante, au cours de laquelle on insère le tube à vide dans le cylindre de protection,
- une étape d'aspiration (210, 410, 610), par le biais de la dépression du tube à vide, du liquide à travers ledit filtre, ledit filtre retenant lesdites cellules et
- une étape de mise en mouvement relatif d'un moyen mobile présentant des pattes et du compartiment pour appliquer une force sur le filtre et libérer le filtre après aspiration du liquide à travers le filtre, les pattes du moyen mobile exerçant, lorsque le doigt d'un opérateur met en mouvement relatif le moyen mobile et le compartiment, une pression sur un support du filtre et libérant le support de filtre de l'ouverture du compartiment.

10. Procédé selon la revendication 9, qui comporte une étape (612) de retrait conjoint du cylindre de protection (502) et du tube à vide (185).

11. Procédé selon l'une quelconque des revendications 8 à 9, dans lequel ledit cylindre de protection (502) comportant une pellicule amovible (510) obturant une ouverture du cylindre de protection opposée au moyen de liaison (504, 520), ledit procédé comporte une étape de retrait de ladite pellicule amovible avant d'insérer le tube à vide dans le cylindre de protection par ladite ouverture avant l'insertion du tube à vide (185) dans le cylindre de protection.

## Patentansprüche

1. Vorrichtung zum Isolieren fixierter oder lebender Zellen auf einem Filter (108, 308) oder zum Extrahieren des genetischen Materials aus den besagten Zellen, folgendes umfassend:
- eine Kammer (102, 302), die ein Innenvolumen aufweist, um eine Flüssigkeit aufzunehmen, in der die besagten Zellen enthalten sind, eine untere Öffnung und einen Lufteinlass,
- ein Mittel (110), das im Verhältnis zur Kammer beweglich ist, wobei dieses bewegliche Mittel Laschen aufweist,
- den Filter, der zumindest vorübergehend fest mit der besagten Öffnung der Kammer oder mit dem beweglichen Mittel verbunden ist, und der sich dazu eignet, die besagten Zellen beim Durchlauf der Flüssigkeit durch den Filter zurückzuhalten, und
- eine Nadel (180), die zumindest vorübergehend fest und in dichter Form mit der besagten Öffnung der Kammer verbunden ist, wobei sich der Filter zwischen der Nadel und dem Innenvolumen der Kammer befindet, wobei sich die besagte Nadel dazu eignet, einen Stopfen (186) einer Vakuumröhre (185) zu durchstechen, die im Verhältnis zum Umgebungsdruck einen Unterdruck aufweist, um die Flüssigkeit durch den besagten Filter anzusaugen,
**dadurch gekennzeichnet, dass** sie darüber hinaus eine Verbindungsvorrichtung (504, 520) zwischen der Kammer und einem Schutzzylinder (502) umfasst, der die Nadel umgibt, wobei sich die besagte Verbindungsvorrichtung dazu eignet, die Vakuumröhre beim Ansaugen der Flüssigkeit durch den Filter zumindest teilweise zu umgeben,
wobei das bewegliche Mittel und die Kammer konfiguriert sind, um zueinander in Bewegung versetzt zu werden, um eine Kraft auf den Filter auszuüben und den Filter nach dem Ansaugen der Flüssigkeit durch den Filter hindurch freizugeben, und die Laschen des beweglichen Mittels dazu konfiguriert sind, um einen Druck auf einen Filterhalter auszuüben und den Filterhalter von der Öffnung der Kammer freizugeben, wenn der Finger eines Bedieners das bewegliche Mittel und die Kammer zueinander in Bewegung versetzt.

2. Vorrichtung nach Anspruch 1, die zumindest vorübergehend den besagten Schutzzylinder (502) umfasst.

3. Vorrichtung nach irgendeinem der Ansprüche 1 oder 2, wobei die besagte Verbindungsvorrichtung (504, 520) provisorisch ist und die gemeinsame Abnahme des Schutzzylinders (502) und der Vakuumröhre (185) ermöglicht.

4. Vorrichtung nach irgendeinem der Ansprüche 1 bis 3, wobei der besagte Schutzzylinder (502) eine abnehmbare Folie (510) umfasst, die eine Öffnung des Schutzzylinders abdeckt, die gegenüber der Verbindungsvorrichtung (504, 520) liegt, und sich die Öffnung zur Einführung der Vakuumröhre (185) in den Schutzzylinder nach dem Abzug der abnehmbaren Folie eignet.

5. Vorrichtung nach irgendeinem der Ansprüche 1 bis 4, wobei der abnehmbare Filterhalter (108, 308) aus Chirurgenstahl gefertigt ist und sich dazu eignet, vorübergehend fest mit der unteren Öffnung der Kammer (102, 302) verbunden zu werden.

6. Vorrichtung nach Anspruch 5, wobei sich die Dicke des besagten Filterhalters (108, 308) dazu eignet, seinen Einsatz in einen Scanner zu ermöglichen.

7. Vorrichtung nach irgendeinem der Ansprüche 5 oder 6, wobei der besagte Filterhalter (108, 308) eine Benutzeridentifizierung trägt.

8. Vorrichtung nach einem der Ansprüche 1 bis 7, die ein abnehmbares Endstück umfasst, das in dichter Form und abnehmbar auf der Kammer befestigt ist, und sich dazu eignet, die Bewegung des beweglichen Mittels und des Abteils zueinander zu unterbinden, durch die es möglich ist, die besagte Kraft anzuwenden und den besagten Filterhalter freizugeben.

9. Verfahren zum Isolieren lebender Zellen auf einem Filter (108, 308) oder zum Extrahieren des genetischen Materials aus den besagten Zellen, welches folgendes umfasst:
- einen Schritt (202, 402, 602) zur zumindest vorübergehenden festen Verbindung eines Filters (108, 308) mit einer unteren Öffnung einer Kammer (102, 302), die darüber hinaus einen Lufteinlass umfasst,
- einen Schritt (208, 405, 604) zum Einfüllen einer Flüssigkeit die besagten Zellen umfassend in die besagte Kammer und
- einen Schritt zur zumindest vorübergehenden festen und dichten Verbindung (202, 402, 602) einer Nadel (180) mit der besagten Öffnung der Kammer, wobei sich der Filter zwischen der Nadel und dem Innenvolumen der Kammer befindet,
**dadurch gekennzeichnet, dass** es darüber hinaus folgendes umfasst:
- einen Schritt (602) zur zumindest vorübergehenden Befestigung eines Schutzzylinders (502) auf der Kammer, wobei der besagte Schutzzylinder dadurch die Nadel umgibt,
- einen Schritt zum Durchstechen (210, 406, 605, 606) mit der besagten Nadel eines Stopfens (186) einer Vakuumröhre (105), die im Verhältnis zum Umgebungsdruck einen Unterdruck aufweist, im Laufe dessen man die Vakuumröhre in den Schutzzylinder einführt,
- einen Schritt zum Ansaugen (210, 410, 610) der Flüssigkeit durch den besagten Filter hindurch, durch den Unterdruck der Vakuumröhre, wobei der besagte Filter die besagten Zellen zurückhält und
- einen Schritt zur Bewegungsversetzung eines beweglichen Mittels mit Laschen und der Kammer zueinander, um eine Kraft auf den Filter auszuüben, und den Filter nach dem Ansaugen der Flüssigkeit durch den Filter hindurch freizugeben, wobei die Laschen des beweglichen Mittels einen Druck auf einen Filterhalter ausüben und den Filterhalter von der Öffnung der Kammer freigeben, wenn der Finger eines Bedieners das bewegliche Mittel und das Abteil zueinander in Bewegung versetzt.

10. Verfahren nach Anspruch 9, das einen Schritt (612) zur gemeinsamen Abnahme des Schutzzylinders (502) und der Vakuumröhre (185) umfasst.

11. Verfahren nach irgendeinem der Ansprüche 8 bis 9, wobei der besagte Schutzzylinder (502) eine abnehmbare Folie (510) umfasst, die eine Öffnung des Schutzzylinders abdeckt, die gegenüber der Verbindungsvorrichtung (504, 520) liegt, und das besagte Verfahren einen Schritt zum Abziehen der besagten abnehmbaren Folie vor dem Einführen der Vakuumröhre (185) in den Schutzzylinder durch die besagte Öffnung vor dem Einführen der Vakuumröhre (185) in den Schutzzylinder umfasst.

## Claims

1. Device for isolating fixed or living cells on a filter (108, 308) or for extracting the genetic material of said cells, which comprises:
- a compartment (102, 302) having an inner volume for receiving a liquid comprising said cells, a lower opening and an air inlet;
- a means (110) mobile relative to the compartment, this mobile means having tabs,
- the filter fixed, at least temporarily, to said opening of the compartment or to said mobile means, and designed to retain said cells as the liquid passes through the filter; and
- a needle (180) hermitically fixed, at least temporarily, to said opening of the compartment, the filter being located between the needle and the inner volume of the compartment, said needle being designed to perforate a plug (186) of a vacuum tube (185) having low pressure relative to the ambient pressure for drawing the liquid through said filter,
**characterized in that** it further comprises a means of linkage (504, 520) between the compartment and a protection cylinder (502) surrounding the needle, said linkage means being designed to surround, at least partially, the vacuum tube during the drawing of the liquid through the filter,
wherein the mobile means and the compartment are configured to be put in relative motion to apply a force on the filter and release the filter after drawing the liquid through the filter, and wherein the tabs of the mobile means are configured to, when an operator's finger puts the mobile means and the compartment in relative motion, to exert a pressure on a mount of the filter and release the filter mount from the opening of the compartment.

2. Device according to claim 1, which comprises, as least temporarily, said protection cylinder (502).

3. Device according to any one of claims 1 or 2, wherein said linkage means (504, 520) is temporary and allows the protection cylinder (502) and the vacuum tube (185) to be withdrawn together.

4. Device according to any one of claims 1 to 3, wherein said protection cylinder (502) comprises a removable film (510) closing an opening of the protection cylinder opposite to the linkage means (504, 520), which opening is designed for introducing the vacuum tube (185) into the protection cylinder after the removable film is withdrawn.

5. Device according to any one of claims 1 to 4, wherein the removable filter mount (108, 308) is made of surgical steel and is designed to be fixed temporarily to the lower opening of the compartment (102, 302).

6. Device according to claim 5, wherein the thickness of said filter mount (108, 308) is designed to allow its passage through a scanner.

7. Device according to any one of claims 5 or 6, wherein said filter mount (108, 308) bears an identifier.

8. Device according to one of claims 1 to 7, which comprises a removable end-fitting fixed to the compartment in a hermitic and removable way and designed to prevent the relative motion of the mobile means and the compartment that allows said force to be applied and said filter mount to be released.

9. Method for isolating fixed or living cells on a filter (108, 308) or for extracting the genetic material of said cells, which comprises:
- a step (202, 402, 602) of fixing, at least temporarily, a filter (108, 308) to a lower opening of a compartment (102, 302) having, in addition, an air inlet;
- a step (208, 405, 604) of inserting a liquid containing said cells into said compartment; and
- a step of fixing (202, 402, 602), at least temporarily, a needle (180) to said opening of the compartment, in a hermetic way, the filter being located between the needle and the inner volume of the compartment,
**characterized in that** it comprises, in addition:
- a step (602) of fixing, at least temporarily, a protection cylinder (502) to the compartment, said protection cylinder thus surrounding the needle;
- a step of perforating (210, 406, 605, 606), with said needle, a plug (186) of a vacuum tube (185) having low pressure relative to the ambient pressure, during which the vacuum tube is inserted into the protection cylinder;
- a step of drawing (210, 410, 610) liquid through said filter by means of the low pressure of the vacuum tube, said filter retaining said cells; and
- a step of putting a mobile means having tabs and the compartment in relative motion to apply a force on the filter and release the filter after drawing the liquid through the filter, the tabs of the mobile means exerting, when an operator's finger puts the mobile means and the compartment in relative motion, a pressure on a mount of the filter and releasing the filter mount from the opening of the compartment.

10. Method according to claim 9, which comprises a step (612) of withdrawing the protection cylinder (502) and the vacuum tube (185) together.

11. Method according to any one of claims 8 to 9, wherein said protection cylinder (502) comprising a removable film (510) closing an opening of the protection cylinder opposite to the linkage means (504, 520), said method comprises a step of withdrawing said removable film before inserting the vacuum tube into the protection cylinder by said opening before the vacuum tube (185) is inserted into the protection cylinder.
